# EUROPEAN PATENT APPLICATION

(11) **EP 4 184 521 A1**
(43) Date of publication of application: **24.05.2023**
(21) Application number: 21209576.4
(22) Date of filing: 22.11.2021
(51) Int. Cl.: G16H 40/40, G16H 10/20, G05B 23/02, G06Q 10/00, G06F 40/20, G07C 3/00, G06F 11/22, G06F 11/07, G06F 9/451

(54) **IDENTIFYING THE CAUSE OF A PROBLEM WITH A MEDICAL IMAGING DEVICE**

(71) Applicant: Koninklijke Philips N.V., 5656 AG Eindhoven (NL)
(72) Inventor: PATIL, Meru Adagouda, Eindhoven (NL); SREENIVASAN, Rithesh, Eindhoven (NL); PAUL, Soubhik, Eindhoven (NL); GAO, Qi, Eindhoven (NL); JOHNSON, Mark Thomas, Eindhoven (NL)
(74) Representative: Philips Intellectual Property & Standards

(57) **Abstract**

A mechanism for generating a technical description of a fault of a medical imaging device. A questionnaire for a respondent is tailored based on at least a user profile of the respondent. The response of the respondent to the questionnaire is used to identify features of the fault, which is in turn used to generate and output the fault description.

## Description

### FIELD OF THE INVENTION

The present invention relates to the field of equipment maintenance, and in particular to the field of identifying the cause or solution of a problem with a medical imaging device.

### BACKGROUND OF THE INVENTION

Medical imaging devices often fail or suffer from one or more issues/problems requiring maintenance. Usually, when a problem is identified in such a device, an individual, known as a respondent, will contact a support operator (e.g. over the telephone or via a text-based service). The support operator will then attempt to diagnose the problem, and propose a solution to resolve the problem. This solution may include replacement of one or more parts of the device, updating of software or any other known approach to repairing a device.

However, a single call or communication session raising an issue is frequently not enough to identify and communicate the problem at hand. There is usually limited time for an individual to explain the issue and such individuals may not be proficient in explaining the problem in detail and/or explaining in with correct technical language. For instance, the individual may be untrained or inexperienced in technical matters pertaining to the medical imaging device. As a result, diagnosing a problem or identifying a solution to the problem often requires multiple calls or communication sessions and/or one or more visits to the site housing the medical imaging device. This leads to delays in fixing of the issue.

In a clinical environment, delays in the availability of a medical imaging device can have a significant impact on the likely health outcome for subjects being treated/assessed in the clinical environment, as any treatment delay can significantly reduce a likelihood of a positive health outcome. In an industrial environment, there is a strong motivation to avoid downtime, in order to maintain production of a product or provision of a service.

There is therefore a desire to reduce the length of time taken to identify the cause or solution to a maintenance issue in a medical imaging device.

### SUMMARY OF THE INVENTION

The invention is defined by the claims.

According to examples in accordance with an aspect of the invention, there is provided a processing system for generating a fault description for a fault of a medical imaging device.

The processing system comprises one or more processors configured to: identify a user profile of a respondent, wherein the user profile indicates a staffing role of the respondent and/or a familiarity of the respondent with the medical imaging device and/or with the operation of the medical imaging device; adapt or generate a fault questionnaire based on at least the identified user profile; receive respondent input data provided by the respondent in response to the adapted fault questionnaire; identify one or more features of the medical imaging device fault, wherein the identifying comprises processing at least an output of a natural language processing analysis performed on the respondent input data; and output a fault description comprising the identified one or more features of the medical imaging device fault.

The proposed approach provides a mechanism for generating a fault description or report of a fault of a medical imaging device. A user profile of an individual (respondent), which indicates or represents how familiar that individual (respondent) is with a medical image device and/or its operation, is used to adapt a fault questionnaire. The responses of the individual to the questionnaire are analyzed and used to generate a fault description.

In this way, the generation of a fault description adapts and responds to an experience level of the individual reporting the fault. More specifically, the questionnaire is adapted, e.g. to ensure that the most appropriate line of questioning of the individual (reporting the fault) is performed, to more accurately predict a possible fault of the medical imaging device.

The one or more processors may be configured to identify the user profile of the respondent by: receiving initial respondent input data provided by a respondent relating to the fault of the medical imaging device; and performing a second natural language processing analysis on the initial respondent input data to identify the user profile of the respondent.

The one or more processors may be configured to identify the one or more features of the medical imaging device fault by performing the natural language processing analysis on at least the initial respondent input data and the respondent input data.

In some examples, the one or more processors are configured to adapt or generate the fault questionnaire by: obtaining a plurality of template fault questionnaires; selecting one of the plurality of template fault questionnaires based on the user profile; and generating the fault questionnaire using the selected template fault questionnaire.

In at least one example, the one or more processors are configured to adapt the fault questionnaire by: receiving initial respondent input data provided by a respondent relating to the fault of the medical imaging device; identifying one or more predicted features of the fault of the medical imaging device by processing at least the initial respondent input data; and adapting the fault questionnaire based on at least the identified user profile and the one or more predicted features of the fault of the medical imaging device.

The one or more predicted features of the fault may include one or more predicted components of the medical imaging device that resulted in the fault.

The one or more processors may be further configured to identify the one or more predicted features by processing at least the initial respondent input data and the user profile.

Optionally, processing the initial respondent input data and the user profile comprises: obtaining device metadata providing information on the medical imaging device; identifying a subset of the device metadata based on the initial respondent input data and the user profile; and identifying one or more predicted features of the fault of the medical imaging device based on the subset of the device metadata.

In some embodiments, identifying a subset of the device metadata based on the initial respondent input data comprises: defining the device metadata as a tree-based structure formed of a plurality of nodes, in which different nodes of the tree-based structure represent different instances of device metadata and connections between nodes represent relationships between the different instances of device metadata; and traversing the tree-based structure based on the initial respondent input data and the user profile to identify contextually relevant instances of device metadata as the subset of the device metadata.

The one or more processors may be configured to identify one or more features of the medical imaging device fault by processing the one or more predicted features of the fault and the output of the natural language processing analysis.

Optionally the one or more processors are configured to identify one or more features of the medical imaging device fault by: defining a dataset containing identifiers of potential features of faults for medical imaging devices; and processing the dataset, using the one or more predicted features of the fault and the output of the natural language processing analysis, to identify one or more features of the medical imaging device fault.

In some examples, the output of the natural language processing analysis comprises one or more keywords or key phrases extracted from at least the respondent input data using the natural language processing analysis.

The one or more processors may be further configured to identify one or more log files of the medical imaging device relating to the fault, based on the respondent input data and optionally the identified user profile.

There is also proposed a computer-implemented method for generating a fault description for a fault of a medical imaging device. The computer-implemented method comprises: identifying a user profile of the respondent, wherein the user profile indicates a staffing role of the respondent and/or a familiarity of the respondent with the medical imaging device and/or with the operation of the medical imaging device; adapting or generating a fault questionnaire based on at least the identified user profile; receiving respondent input data provided by the respondent in response to the adapted fault questionnaire; identifying one or more features of the medical imaging device fault, wherein the identifying comprises processing at least an output of a natural language processing analysis performed on the respondent input data; and outputting a fault description comprising the identified one or more features of the medical imaging device fault.

There is also proposed a computer program product comprising computer program code means which, when executed on a computing device having a processing system, cause the processing system to perform all of the steps of any herein described method.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates an approach according to an embodiment;
Fig. 2 illustrates a process for use in an approach;
Fig. 3 conceptually illustrates a fault questionnaire;
Fig. 4 conceptually illustrates a knowledge graph;
Fig. 5 illustrates an approach for generating a technical description;
Fig. 6 illustrates another approach for generating a technical description; and
Fig. 7 illustrates a processing system according to an embodiment.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for generating a technical description of a fault of a medical imaging device. A questionnaire for a respondent is tailored based on at least a user profile of the respondent. The response of the respondent to the questionnaire is used to identify features of the fault, which is in turn used to generate and output the fault description.

Embodiments are based on the realization that a more appropriate questionnaire for a respondent can be provided by configuring the questionnaire based on information about the respondent. By configuring the questionnaire in this way, more useful information can be acquired from the respondent to generate a technical description with more accuracy.

Here described approaches can be employed in any support system for handling errors in medical imaging devices. Embodiments are particularly advantageous when employed to aid support operators in diagnosing and identifying faults in medical imaging devices, as reported by a (typically less technically proficient) respondent.

The skilled person would be readily capable of developing a processing system for carrying out any herein described method. Thus, each step of any illustrated flowchart may represent a different action performed by a processing system, and may be performed by a respective module of the processing system.

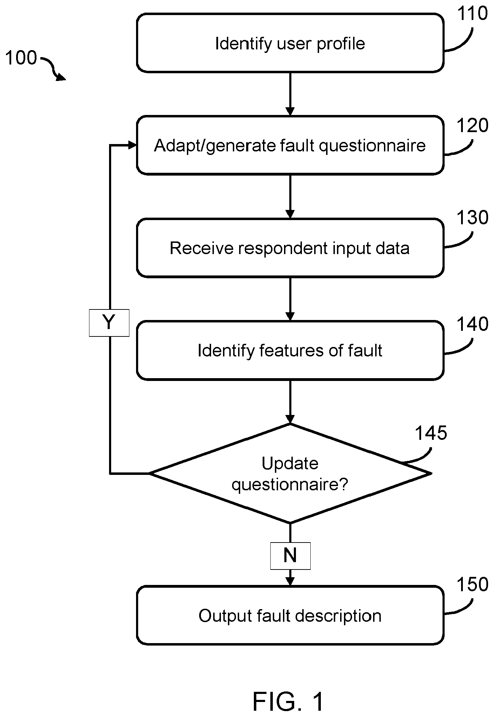

Fig. 1 is a flowchart illustrating a method 100 according to an embodiment. The method 100 is configured for generating a fault description for a fault of a medical imaging device (reported by a respondent).

The method 100 comprises a step 110 of identifying a user profile of the respondent. The user profile indicates a staffing role of the respondent and/or a familiarity of the respondent with the medical imaging device and/or with the operation of the medical imaging device.

The user profile therefore provide information on the characteristics of the user, in particular, characteristics that might influence with which medical imaging device or parts of a medical imaging device they interact with or characteristics that affect a level of technical expertise and language usable by the respondent to identify and/or describe information about a fault.

The user profile might also include additional information about other suitable characteristics of the respondent, such as their working environment (e.g. name of the clinical environment) and so on.

The user profile may be retrieved or obtained from a user profile database. In such examples, a user identifier (such as a name of the respondent or an identifier of the communication device use by the respondent, such as a cellular phone number) is used to identify the appropriate user profile in the user profile database. The name of the respondent may be identified by performing a natural language processing technique on initial text provided by the respondent.

Another approach could be used to determine or generate the user profile, e.g. if the user profile does not exist in the user profile database or cannot be otherwise identified. This approach may comprise a process of providing one or more profiling questions for a respondent, each profiling question requesting information about certain characteristics of the user. The response of the respondent to profiling questions may be used to define/generate the user profile. The profiling questions may, for example, be of a general nature to facilitate identification of suitable characteristics of the user (for example job description, study direction).

The generated user profile for the respondent could then be added to the user profile database, if present.

The method 100 then performs a step 120 of adapting or generating a fault questionnaire based on at least the identified user profile. In this way, the user profile is used to define the fault questionnaire, so that different fault questionnaires are provided for different user profiles and/or re spondents.

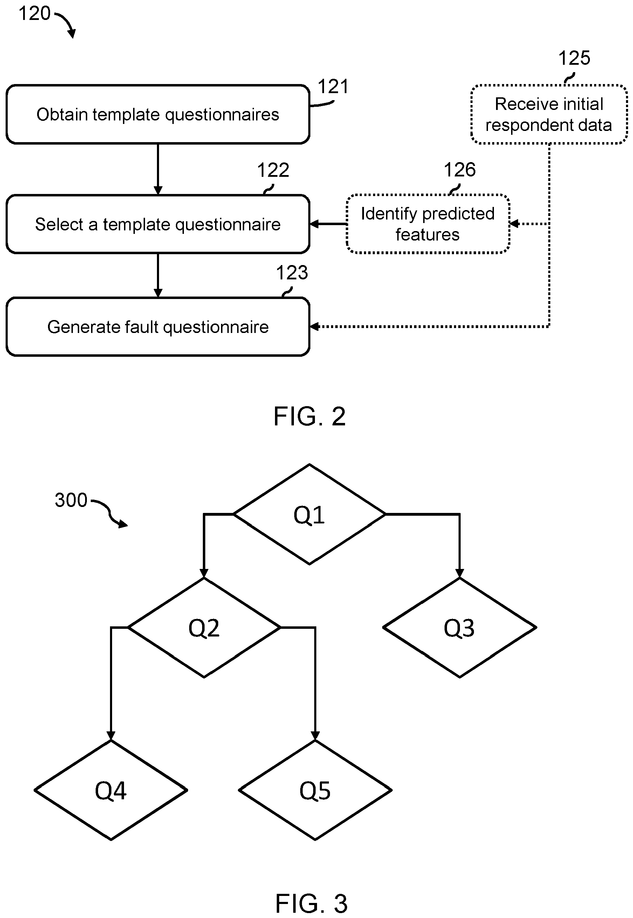

Fig. 2 illustrates one approach for performing step 120 of method 100.

In this example, the step 120 comprises a sub-step 121 of obtaining a plurality of template fault questionnaires. Each template may define a different sequence or series of questions about a potential fault for the respondent to answer. Different templates may provide different questions at different levels of technical expertise, medical expertise, imaging device familiarity and so on.

The step 120 further comprises a sub-step 122 of selecting one of the plurality of template fault questionnaires based on the user profile.

In this way, different user profiles or types of user profile will be associated with different templates. In particular, if the user profile identifies a staff role of the respondent, a particular template may be selected based on the staff role.

For instance, if the user profile indicates the respondent is a technician, then a template associated with a technician will be selected in sub-step 122. A template for a technician can start with a workflow or operating process, e.g. requesting if the system has been slower in response for particular type of scan or the like.

As another example, if the respondent is an engineer then a corresponding engineer questionnaire template should be selected in sub-step 122. A template for a bio-med or in-house service engineer may start with one or more questions about technical aspect like "when was last planned maintenance carried out?", or requesting information about pressure readings of a gauge under discussion.

As another example, if the respondent is a clinician then a corresponding clinician questionnaire template should be selected. A template for clinician may begin with clinically relevant questions, e.g. with respect to image quality of an image provided by the medical imaging device.

In this way, different templates can be targeted to different specialties or roles of the respondent.

It will be appreciated that these provide only a small sample of possible templates that could be selected based on information in a user profile. For example, a "clinician" template could be sub-divided into different types of template, such as providing one template for radiologists, another for nurses and different one for oncologists.

The above description provides examples for use where the user profile defines a role of the user. However, the user profile may contain other/additional information about characteristics of the user, and different templates may be associated with different combinations of any characteristics included in the user profile.

As another example, if the user profile defines an experience level, different templates may be associated with different experience levels. In some embodiments, different templates may be associated with respondents of a same role, but different experience levels (where different templates are still associated with respondents of different roles).

In some examples, other information is used in addition to the user profile to select the template questionnaire.

For instance, step 120 may be configured to further comprise a step 125 of receiving initial respondent input data provided by a respondent relating to the fault of the medical imaging device. Step 120 may further comprise a step 126 of identifying one or more predicted features of the fault of the medical imaging device by processing at least the initial respondent input data.

In this example, the sub-step 122 may be configured to select the template questionnaire based on at least the identified user profile and the one or more predicted features of the fault of the medical imaging device.

The initial respondent data may, for instance, contain information identifying the type of medical device, initial information on a problem with the device and so on. This initial respondent data can therefore provide useful contextual information for predicting features of the fault of the medical device. Predicted features might include, for example, information identifying an identifier of the medical device (or a sub-system of the medical device) associated with the fault, an event that caused or uncovered the fault and so on.

Approach for identifying predicted features by processing respondent input data is provided later in this disclosure. Such approaches may, as later described, also be adapted to make use of the user profile to identify the predicted feature(s).

Different template fault questionnaires may be associated with different predicted features of the fault, e.g. different types of medical devices, different sub-systems of a medical device or different events that uncovered the fault. The template may be designed in a manner that it would capture exhaustively all questions related to a particular feature (e.g. a particular sub-system).

Thus, for example, a first template fault questionnaire may be used for a first type of medical imaging device (e.g. a CT scanner), and a second template fault questionnaire may be used for a second type of medical imaging device (e.g. a MRI scanner). More granularity could be achieved by providing different template fault questionnaires for different sub-systems of particular medical imaging devices.

A combination of the user profile and the other information (here: any predicted features) may be used to select a template fault questionnaire. Accordingly, different template fault questionnaires may be associated with different combinations of user profile information and other information.

The step 120 then moves to a sub-step 123 of generating the fault questionnaire using the selected template fault questionnaire.

In some examples, the selected template fault questionnaire may itself define the generated fault questionnaire. Put another way, a template fault questionnaire may simply comprise a predefined series of specific questions for establishing information about the fault.

In other embodiments, additional information may be used to generate the selected fault questionnaire. For example, a template questionnaire may comprise one or more question templates, being questions having slots that can be completed or filled out (e.g. based on additional information). Example slots might include "device" (which can be filled out with information about the medical device), "user" (which can be filled out with information about a subject involved with the device") and "feature" (which can be filled out with information about sub-systems, modalities or expected operations/capabilities of the medical imaging device).

As one example, additional information may comprise information about the medical device (e.g. an identifier of the medical device). The slots in the questionnaire may be completed or filled out based on the information about the medical device. For instance, a database may (for different medical devices) provide different data to fill the slots of a template questionnaire.

Thus, an example slot for a "device" - e.g. in the question template, "Was the DEVICE in clinical use at the time the issue was discovered?" - could be replaced or filled with the name or identifier of the medical device (e.g. DEVICE can be replaced with "CT scanner" if the medical imaging device is a CT scanner).

As another example, an example slot for a "user" - e.g. in the question template "Was any USER harmed?" - could be replaced or filled with an identifier of an expected user or subject of the medical device (e.g. USER could be replaced with "patient" if the medical imaging device is aCT scanner for scanning a patient).

It has previously been described how, step 120 may be configured to further comprise a step 125 of receiving initial respondent input data provided by a respondent relating to the fault of the medical imaging device. Based on information contained in the initial respondent input data, slots in the template questionnaire may be completed. This may include information on protocols, parts, image artefacts, scan type, user, features, parameters, etc.

The fault questionnaire may be updated based on feedback from the respondent, e.g. based on a response of the respondent to questions of the questionnaire. In this way, the question flow can be dynamically changed based on response from the respondent.

Thus, the fault questionnaire may be iteratively updated based on respondent input data provided by the respondent in response to the fault questionnaire.

In some examples, the fault questionnaire effectively acts as a flow chart, where different responses of the respondent change a path through the flow chart (e.g. causes different questions to be asked).

Fig. 3 conceptually illustrates a portion of such a fault questionnaire 300, which illustrates 5 questions Q1 - Q5 forming part of the fault questionnaire. Different responses (e.g. affirmative or negative) to a question of the fault questionnaire will define the next question for the respondent.

In some examples of fault questionnaires, questions may be at different levels of detail. In particular, if a respondent provides affirmative/appropriate/sufficient information at a more general level of detail, then the next question may be at a more detailed and precise level of detail. Thus, if a respondent for the questionnaire continues to answer questions affirmatively/appropriately/sufficiently, the, the flow through successive questions in the questionnaire may be more detailed and precise than preceding questions.

On the other hand, the fault questionnaire may also be set up so that, for certain questions, if not answered properly, then the questioning will be redirected to a higher level element.

In this way, the questions provided to the respondent using the fault questionnaire may evolve responsive to the response of the respondent to the questions.

Turning back to Fig. 1, the method 100 comprises a step 130 of receiving respondent input data provided by the respondent in response to the adapted or generated fault questionnaire.

Thus, the fault questionnaire is presented to the respondent, e.g. automatically or via a discussion with a support operator, and the reply of the respondent to the fault questionnaire is obtained. Presenting the fault questionnaire to the respondent may comprise providing successive questions to the respondent so as to move through the fault questionnaire, e.g. from a lower level of detail to a greater level of detail. The responses of the respondent to the questions may change a flow or movement through the fault questionnaire.

Step 130 may therefore comprise a step of providing questions to the respondent or support operator (to ask the respondent) based on the fault questionnaire, and obtaining respondent input data provided by the respondent (e.g. via the support operator) in response to the questions. The questions provided to the respondent may be defined by a flow through the fault questionnaire, which may change responsive to the responses of the subject. Of course, the fault questionnaire may be updated based on the responses of the subject during the questioning.

The method 100 further comprises a step 140 of identifying one or more features of the medical imaging device fault, wherein the identifying comprises processing at least an output of a natural language processing analysis performed on the respondent input data.

The natural language processing analysis performed in step 140 thereby acts to identify features or characteristics of the medical imaging device fault from input data provided by the respondent. This approach may effectively treat the respondent input data as a generic description of the fault, and use this recognition to identify more specific or precise characteristics of the fault.

In one example, the natural language processing analysis is configured to extract or identify keywords from the respondent input data. The extracted keywords may act as the one or more features of the medical imaging device fault. This embodiment recognizes that information about the medical imaging device fault will be present in the respondent input data, such that extracted keywords can act as features of the medical imaging device fault.

Another approach for performing step 140 is to map the respondent input data to a set of specific components or sub-system of a device in order to identify probable areas in which the fault has occurred. This approach may make use of a knowledge graph, which is a data structure representing the system as a set of sub-systems and components and the relationships therebetween. Each sub-system and/or component may be associated with a set of keywords. The natural language processing analysis may use keyword matching to narrow down the probable sub-system/components from a set of sub-systems/components in the knowledge graph. The knowledge graph can be created using design and requirement documentation of the system. This documentation may have hierarchical organization from device (system) level to sub-systems and individual components. A traceability document can be used to map and connect nodes in this knowledge graph.

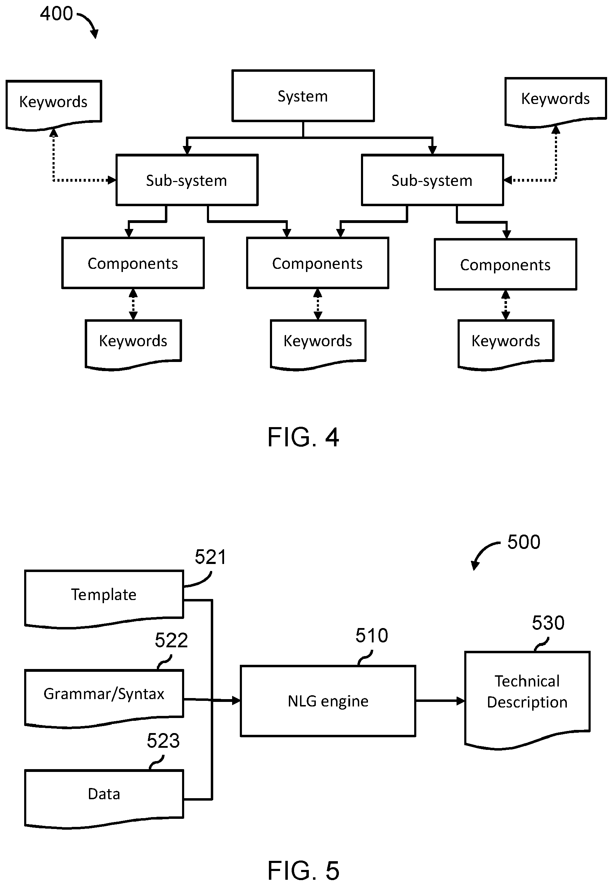

Fig. 4 provides a representative illustration of a knowledge graph 400, which indicates relationships and links between components, sub-systems and keywords. It will be appreciated how keywords can be associated with particular components and/or sub-systems, so that keyword matching can be performed to identify the most probable sub-systems and/or keywords associated with the fault from the respondent input data.

In some examples, narrowing down of the probable sub-system or components can additionally or alternatively be performed using the user profile. For instance, if a user profile indicates a role of the respondent, then a role may be used to narrow down the sub-system and/or components. For instance, persons of different roles may only be capable of making use of certain systems or sub-systems. For instance, a technician may only be trained to make use of a certain sub-system or modality. A role based context database may be maintained for this purpose.

Thus, one or more sub-systems and/or components of the medical imaging device (which are predicted to relate to the fault) can be identified using the identified user profile and/or the respondent input data.

Step 140 may then use the respondent input data, and optional additional data, to generate, for each of a plurality of possible issues within the narrowed down sub-systems/components, a probability score that said issue has occurred. As one example, an AI based classification engine could be used to predict the above-mentioned probabilities by processing at least keywords extracted from the respondent input data by the natural language processing analysis mechanism. A probability score can thereby be generated for each such possible issue diagnosis. The probability score(s) can act as the identified one or more features of the medical imaging device fault.

The AI-based classification engine can be rule based or can be a machine-learning or deep-learning model trained on previously diagnosed case histories. Such a machine-learning or deep-learning model may be trained based on historical data of keywords extracted from historical respondent input data and corresponding fault diagnoses (e.g. from a site visit).

It has been mentioned how additional data could be used to generate the probability score(s).

This additional data may include, for instance, metadata and/or log data of the medical imaging data. The metadata and/or log data may be determined by using the narrowed down sub-systems/components to identify which metadata and/or log data (from a larger dataset for the medical imaging system) to obtain. Thus, the metadata and/or log data may be determined based on the identified user profile and/or the respondent input data.

In some examples, time information may be used to define the relevant metadata and/or log data. The time information may indicate the time or time period when the issue occurred. The time information may be extracted, for instance, from respondent input data (e.g. by the respondent indicating when the error occurred).

As another example, the time information may be identified by identifying a sub-system or sub-component that caused the issue and identifying, in the larger dataset of metadata and/or log data of the medical imaging system, the occurrence of an error - and identifying the time at which the error occurred as that time information.

Of course, it is not essential to use time information. Rather, any errors in the relevant metadata and/or log data of the specific identified sub-component could be identified and extracted.

In some examples, the metadata may be obtained by obtaining a dataset of metadata for the device; identifying a subset of the device metadata based on the initial respondent input data and the user profile; and identifying one or more predicted features of the fault of the medical imaging device based on the subset of the device metadata.

In particular, obtaining the metadata based on the initial respondent input data and the user profile may comprise: defining the device metadata as a tree-based structure formed of a plurality of nodes, in which different nodes of the tree-based structure represent different instances of device metadata and connections between nodes represent relationships between the different instances of device metadata; and traversing the tree-based structure based on the initial respondent input data and the user profile to identify contextually relevant instances of device metadata as the subset of the device metadata.

In some examples, the additional data could include historic information about the medical imaging device, e.g. a repair history. This approach recognizes that previous repairs or the history of a device influences a likely ongoing fault of the medical imaging device.

For improved performance of step 120, the probability score(s) could also be used in the generation/adaptation of the fault questionnaire. In particular examples, the probability score(s) could be used to rank the most likely faults, and direct the questions of the questionnaire to unveiling more information about the most probable faults.

For instance, a questionnaire template may be selected based on the most probable fault of the medical imaging device, as in indicated in the probability score(s).

As another example, question templates may be completed or filled based on the most probable fault of the medical imaging device. For instance, different slot types for a question can be filled with appropriate values from a subsystem associated with the most probable fault.

This effectively means that the fault questionnaire may be iteratively updated responsive to a respondent's response to the fault questionnaire, in the form of the respondent input data. Method 100 may therefore further comprise a determination step 145 of whether to update the questionnaire (e.g. based on the respondent input data). A positive determination (which reverts back to step 120) may be performed responsive to the probability of the most probable fault falling below some predetermined threshold. A negative determination (which moves onto a step 150) may be performed responsive to the probability of the most probable fault reaching or breaching some predetermined threshold

The method 100 further comprises the step 150, which comprises outputting a fault description comprising the identified one or more features of the medical imaging device fault. In particular, step 150 may comprise providing a user-perceptible output, e.g. at a user interface, of the fault description. The user-perceptible output may be for the respondent and/or for a support operator.

Step 150 may comprise generating a technical description of the fault. This may be performed by constructing a technical description from the identified one or more features of the medical imaging device fault. A generated technical description may then be output as the fault description.

One approach for generating a technical description could be to use a natural language generation system that takes, as input, the identified one or more features of the medical imaging device fault, as input and converts them to a meaningful description of the actual problem based on, for instance, a pre-defined reporting template.

Fig. 5 illustrates one approach 500 for generating a technical description. In this approach, a natural language generation engine 510 may take (as input) standard templates of technical issue description ("description templates") 521, syntax and/or grammatical rules 522 and the identified one or more features of the medical imaging device fault 523. Techniques such as TF-IDF (term frequency-invert document frequency) can be used by the natural language generation engine 510 to determine which of the identified features 523 are best suited for a given description template 521. Subsequently, the identified features may be organized and structured to create the technical issue description 530 according to the syntax and/or grammatical rules 522.

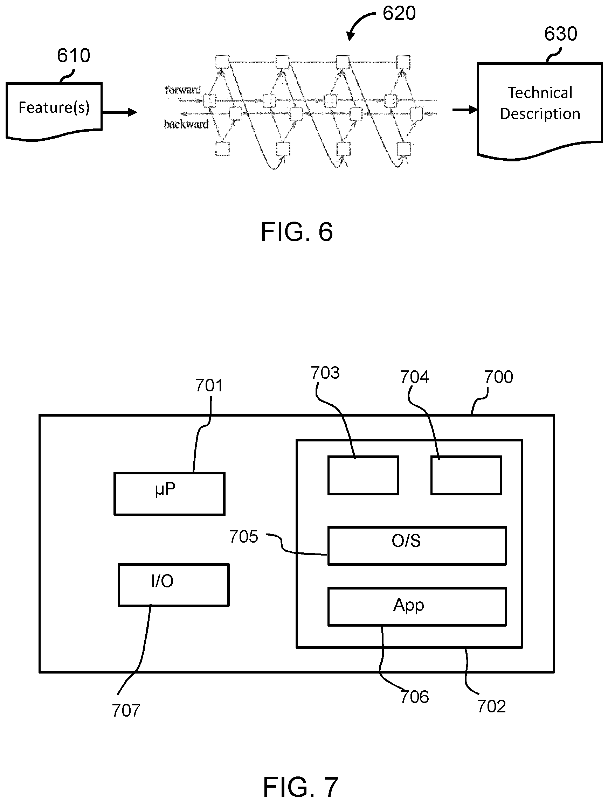

Fig. 6 illustrates an alternative approach 600, which is to perform dynamic generation of sentences in which a natural language generation system is built using a machine-learning or deep-learning model 620 (e.g. RNN, LSTM, Markov chain). In this scenario, the machine-learning or deep-learning model is trained using a dataset for which input data comprises identified one or more features 610 of the medical imaging device fault and the output data is a corresponding technical issue description 630.

If obtained, any extracted log data may also prove useful for a support operator, to allow them to more specifically assess potential causes of a fault. Thus, in some examples, extracted log data is provided as output, e.g. to the support operator. Step 150 may thereby comprise providing a user-perceptible output, e.g. at a user interface, of any extracted log data.

The skilled person would be readily capable of preparing a method for carrying out any herein described approach. Accordingly, there may be a (computer-implemented) method for carrying out any herein described approach.

Similarly, any herein described approach can be performed by an appropriately configured processing system, which would be readily apparent to the skilled person. Thus, each step of the flow chart may represent a different action performed by a processing system, and may be performed by a respective module of the processing system.

By way of example, Fig. 7 illustrates an example of a processing system 700 within which one or more parts of an embodiment may be employed. Various operations discussed above may utilize the capabilities of the processing system 700. For example, one or more parts of a system for generating a fault description for a fault of a medical imaging device may be incorporated in any element, module, application, and/or component discussed herein. In this regard, it is to be understood that system functional blocks can run on a single computer or may be distributed over several computers and locations (e.g. connected via internet).

The processing system 700 includes, but is not limited to, PCs, workstations, laptops, PDAs, palm devices, servers, storages, and the like. Generally, in terms of hardware architecture, the processing system 700 may include one or more processors 701, memory 702, and one or more I/O devices 707 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 701 is a hardware device for executing software that can be stored in the memory 702. The processor 701 can be virtually any custom made or commercially available processor, a central processing unit (CPU), a digital signal processor (DSP), or an auxiliary processor among several processors associated with the processing system 700, and the processor 701 may be a semiconductor based microprocessor (in the form of a microchip) or a microprocessor.

The memory 702 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 702 may incorporate electronic, magnetic, optical, and/or other types of storage media. Note that the memory 702 can have a distributed architecture, where various components are situated remote from one another, but can be accessed by the processor 701.

The software in the memory 702 may include one or more separate programs, each of which comprises an ordered listing of executable instructions for implementing logical functions. The software in the memory 702 includes a suitable operating system (O/S) 705, compiler 704, source code 703, and one or more applications 706 in accordance with exemplary embodiments. As illustrated, the application 706 comprises numerous functional components for implementing the features and operations of the exemplary embodiments. The application 706 of the processing system 700 may represent various applications, computational units, logic, functional units, processes, operations, virtual entities, and/or modules in accordance with exemplary embodiments, but the application 706 is not meant to be a limitation.

The operating system 705 controls the execution of other computer programs, and provides scheduling, input-output control, file and data management, memory management, and communication control and related services. It is contemplated by the inventors that the application 706 for implementing exemplary embodiments may be applicable on all commercially available operating systems.

Application 706 may be a source program, executable program (object code), script, or any other entity comprising a set of instructions to be performed. When a source program, then the program is usually translated via a compiler (such as the compiler 704), assembler, interpreter, or the like, which may or may not be included within the memory 702, so as to operate properly in connection with the O/S 705. Furthermore, the application 706 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 707 may include input devices such as, for example but not limited to, a mouse, keyboard, scanner, microphone, camera, etc. Furthermore, the I/O devices 707 may also include output devices, for example but not limited to a printer, display, etc. Finally, the I/O devices 707 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 707 also include components for communicating over various networks, such as the Internet or intranet.

If the processing system 700 is a PC, workstation, intelligent device or the like, the software in the memory 702 may further include a basic input output system (BIOS) (omitted for simplicity). The BIOS is a set of essential software routines that initialize and test hardware at startup, start the O/S 705, and support the transfer of data among the hardware devices. The BIOS is stored in some type of read-only-memory, such as ROM, PROM, EPROM, EEPROM or the like, so that the BIOS can be executed when the processing system 700 is activated.

When the processing system 700 is in operation, the processor 701 is configured to execute software stored within the memory 702, to communicate data to and from the memory 702, and to generally control operations of the processing system 700 pursuant to the software. The application 706 and the O/S 705 are read, in whole or in part, by the processor 701, perhaps buffered within the processor 701, and then executed.

When the application 706 is implemented in software it should be noted that the application 706 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 706 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method. In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. A processing system (700) for generating a fault description for a fault of a medical imaging device, the processing system comprising one or more processors configured to:
identify (110) a user profile of a respondent, wherein the user profile indicates a staffing role of the respondent and/or a familiarity of the respondent with the medical imaging device and/or with the operation of the medical imaging device;
adapt (120) or generate a fault questionnaire based on at least the identified user profile;
receive (130) respondent input data provided by the respondent in response to the adapted fault questionnaire;
identify (140) one or more features of the medical imaging device fault, wherein the identifying comprises processing at least an output of a natural language processing analysis performed on the respondent input data; and
output (145) a fault description comprising the identified one or more features of the medical imaging device fault.

2. The processing system of claim 1, wherein the one or more processors are configured to identify the user profile of the respondent by:
receiving initial respondent input data provided by a respondent relating to the fault of the medical imaging device; and
performing a second natural language processing analysis on the initial respondent input data to identify the user profile of the respondent.

3. The processing system of claim 2, wherein the one or more processors are configured to identify the one or more features of the medical imaging device fault by performing the natural language processing analysis on at least the initial respondent input data and the respondent input data.

4. The processing system of any of claims 1 to 3, wherein the one or more processors are configured to adapt or generate the fault questionnaire by:
obtaining a plurality of template fault questionnaires;
selecting one of the plurality of template fault questionnaires based on the user profile; and
generating the fault questionnaire using the selected template fault questionnaire.

5. The processing system of any of claims 1 to 4, wherein the one or more processors are configured to adapt the fault questionnaire by:
receiving initial respondent input data provided by a respondent relating to the fault of the medical imaging device;
identifying one or more predicted features of the fault of the medical imaging device by processing at least the initial respondent input data; and
adapting the fault questionnaire based on at least the identified user profile and the one or more predicted features of the fault of the medical imaging device.

6. The processing system of claim 5, wherein the one or more predicted features of the fault include one or more predicted components of the medical imaging device that resulted in the fault.

7. The processing system of claim 5 or 6, wherein the one or more processors are further configured to identify the one or more predicted features by processing at least the initial respondent input data and the user profile.

8. The processing system of claim 7, wherein the processing the initial respondent input data and the user profile comprises:
obtaining device metadata providing information on the medical imaging device;
identifying a subset of the device metadata based on the initial respondent input data and the user profile;
identifying one or more predicted features of the fault of the medical imaging device based on the subset of the device metadata.

9. The processing system of claim 7, wherein the identifying a subset of the device metadata based on the initial respondent input data comprises:
defining the device metadata as a tree-based structure formed of a plurality of nodes, in which different nodes of the tree-based structure represent different instances of device metadata and connections between nodes represent relationships between the different instances of device metadata; and
traversing the tree-based structure based on the initial respondent input data and the user profile to identify contextually relevant instances of device metadata as the subset of the device metadata.

10. The processing system of any of claims 1 to 9, wherein the one or more processors are configured to identify one or more features of the medical imaging device fault by processing the one or more predicted features of the fault and the output of the natural language processing analysis.

11. The processing system of claim 10, wherein the one or more processors are configured to identify one or more features of the medical imaging device fault by:
defining a dataset containing identifiers of potential features of faults for medical imaging devices; and
processing the dataset, using the one or more predicted features of the fault and the output of the natural language processing analysis, to identify one or more features of the medical imaging device fault.

12. The processing system of any of claims 1 to 11, wherein the output of the natural language processing analysis comprises one or more keywords or key phrases extracted from at least the respondent input data using the natural language processing analysis.

13. The processing system of any of claims 1 to 12, wherein the one or more processors are further configured to identify one or more log files of the medical imaging device relating to the fault, based on the respondent input data and optionally the identified user profile.

14. A computer-implemented method (100) for generating a fault description for a fault of a medical imaging device, the computer-implemented method comprising:
identifying (110) a user profile of the respondent, wherein the user profile indicates a staffing role of the respondent and/or a familiarity of the respondent with the medical imaging device and/or with the operation of the medical imaging device;
adapting (120) or generating a fault questionnaire based on at least the identified user profile;
receiving (130) respondent input data provided by the respondent in response to the adapted fault questionnaire;
identifying (140) one or more features of the medical imaging device fault, wherein the identifying comprises processing at least an output of a natural language processing analysis performed on the respondent input data; and
outputting (150) a fault description comprising the identified one or more features of the medical imaging device fault.

15. A computer program product comprising computer program code means which, when executed on a computing device having a processing system, cause the processing system to perform all of the steps of the method according to claim 14.
